(19) Europäisches Patentamt
European Patent Office
Office européen des brevets

(11) EP 3 685 863 B1

(12) EUROPEAN PATENT SPECIFICATION

(45) Date of publication and mention of the grant of the patent:
**26.10.2022 Bulletin 2022/43**

(21) Application number: **20153034.2**

(22) Date of filing: **22.01.2020**

(51) International Patent Classification (IPC):
***A61L 15/34*** (2006.01) ***A61L 15/46*** (2006.01)
***A01N 37/02*** (2006.01) ***A61K 9/00*** (2006.01)
***A61K 31/22*** (2006.01) ***A61K 47/14*** (2017.01)
***A61K 47/44*** (2017.01) ***A01N 37/36*** (2006.01)
***A61F 13/00*** (2006.01) ***A61P 31/04*** (2006.01)

(52) Cooperative Patent Classification (CPC):
(C-Sets available)
**A61P 31/04; A01N 37/36; A61F 13/00063; A61K 31/22; A61L 15/46;** A61L 2300/21 (Cont.)

(54) **BACTERIOSTATIC COMPOSITION AND WOUND DRESSING INCLUDING THE SAME**

BAKTERIOSTATISCHE ZUSAMMENSETZUNG UND DIESE ENTHALTENDER WUNDVERBAND

COMPOSITION BACTÉRIOSTATIQUE ET PANSEMENT POUR PLAIES LA COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.01.2019 IT 201900000887**

(43) Date of publication of application:
**29.07.2020 Bulletin 2020/31**

(73) Proprietor: **Farmamed S.r.l.**
**46034 Borgo Virgilio Mantova (IT)**

(72) Inventors:
- **ANGELINETTA, Kevin**
  **46034 Borgo Virgilio Mantova (IT)**
- **ANGELINETTA, David**
  **46034 Borgo Virgilio Mantova (IT)**
- **ANGELINETTA, Dylan**
  **46034 Borgo Virgilio Mantova (IT)**

(74) Representative: **Biggi, Cristina**
**Bugnion S.p.A.**
**Viale Lancetti 17**
**20158 Milano (IT)**

(56) References cited:
**US-A- 4 540 567** **US-A1- 2005 019 355**
**US-A1- 2014 377 331** **US-A1- 2016 143 825**
**US-A1- 2018 168 870**

- **BIDLAS E ET AL: "Comparing the antimicrobial effectiveness of NaCl and KCl with a view to salt/sodium replacement", INTERNATIONAL JOURNAL OF FOOD MICROBIOLOGY, ELSEVIER BV, NL, vol. 124, no. 1, 10 May 2008 (2008-05-10), pages 98-102, XP022650510, ISSN: 0168-1605, DOI: 10.1016/J.IJFOODMICRO.2008.02.031 [retrieved on 2008-03-07]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

(52) Cooperative Patent Classification (CPC): (Cont.)

C-Sets
**A01N 37/36, A01N 25/02, A01N 25/30**

## Description

The present invention relates to a composition with antibacterial action for the production of bacteriostatic gauzes and patches.

## PRIOR ART

Resident microbial flora, i.e. the set of microorganisms that generally populate the skin, does not have a pathogenic character, but considering the enormous quantity of microorganisms it comes into contact with, the skin can also temporarily host a transient flora, whose components include pathogenic or potentially pathogenic species.

Factors such as diet, hygienic conditions, atmospheric pollution, personal habits and the composition and quantity of sebum and sweat present on the skin influence the type of resident and transient flora of a given individual.

Under normal conditions, the skin possesses instruments of defence that prevent the extensive colonisation thereof by potentially harmful transient bacterial flora.

For example, epithelial cells are periodically renewed and, when they flake off, they carry with them the microbes that settle in the cracks interposed between horny flakes.

Cutaneous lipids, together with sodium chloride and immunoglobulins present in sweat contribute to making the skin an inhospitable environment for a large majority of microbes.

Furthermore, the residential flora hinders colonisation of the skin by pathogens by taking away nourishment from them, producing antimicrobial substances and lowering the value of the skin's pH to levels that are toxic for pathogenic microorganisms, thanks to the degradation of the sebum on which they feed.

Generally, therefore, pathogenic microorganisms do not pass through the natural barriers, except in the case of immunodeficiency or skin lesions.

In the presence of a wound, be it accidental or voluntary, for example in the case of surgical interventions, the entry of microbial cells into the epidermis or dermis offers an opportunity for infection.

In these situations, a radical change takes place in the environmental conditions of the skin. The presence of moisture and necrotic tissue, for example, favours the proliferation of gram-negative pathogens, while hindering the growth of the gram-positive saprophytes that are at the basis of the residential skin flora.

Prompt recognition of a wound infection enables the application of appropriate antimicrobial therapies, which are generally systemic since the infection always interrupts the normal wound healing process.

It thus appears evident that preventing the onset of infections in skin lesions is of primary importance in order to reduce the onset of critical conditions requiring a pharmacological intervention. In the prior art antibacterial compositions are described in US2005/0019355, which discloses compositions comprising 2-50% by volume of an alkyl ester of lactic acid.

In this context, there is thus a felt need to have medical devices capable not only of forming a physical barrier as protection for skin lesions, but also of limiting or preventing the onset of microbial infections.

The primary task of the present invention is therefore to provide new compositions with an antibacterial action that can be used in the preparation of a wound dressing, such as, for example, patches, gauzes and bandages, with an antibacterial action capable of limiting and/or preventing bacterial proliferation on wounds, thus limiting and/or preventing the onset of infections.

A further object of the invention is to provide a process for preparing an antibacterial wound dressing whose production is simple and rapid and entails low costs.

## SUMMARY OF THE INVENTION

The present invention relates to an antibacterial composition comprising:

(a) 2-50% by weight of a compound of formula (I)

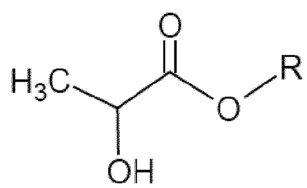

(I)

wherein
R is a linear or branched noncyclic alkyl radical with from 1 to 5 carbon atoms;
(b) 0.9-5.5% by weight of at least one salt selected in the group consisting of NaCl, KCl and mixtures thereof;
(c) 1-10% by weight of at least one ester selected in the group consisting of polyglycerol esters with at least one fatty acid, esters of polyethylene glycol glycerol ethers with at least one fatty acid, and mixtures thereof;
(d) water to 100% by weight.

The invention also relates to a process for preparing an antibacterial material comprising the steps of:

(i) preparing an antibacterial composition as described above;
(ii) optionally preparing a composition comprising at least one tissue regeneration promoting agent;
(iii) optionally mixing the compositions of steps (i) and (ii);
(iv) impregnating an absorbent material with 2-15 g/$m^2$ of the composition of step (i) or step (iii); and
(v) reducing the free water content in the absorbent material to a value less than or equal to 0.5% by weight relative to the total weight of the material.

Furthermore, the invention relates to an antibacterial dressing for skin wounds comprising at least one layer comprising the antibacterial material obtained from the above-described process.

## DETAILED DESCRIPTION OF THE INVENTION

In the context of the present invention, the term "fatty acids" refers to saturated or unsaturated aliphatic acyclic monocarboxylic or polycarboxylic acids with from 4 to 28 carbon atoms in the chain, preferably with an even number of carbon atoms.
In the context of the present invention, the term "(trans)esterification" refers to an esterification and/or transesterification reaction.
In the context of the present invention, INCI names are used to identify the polyglycerol esters and esters of polyethylene glycol glycerol ethers with at least one fatty acid.
In the context of the present invention, the term "antibacterial" is synonymous of bactericidal and/or bacteriostatic.
In the context of the present invention, the percentages are understood as expressed by weight, unless specified otherwise.
In a first aspect, the present invention relates to an antibacterial composition comprising:

(a) 2-50% by weight of at least one compound of formula (I)

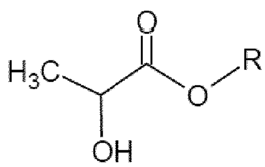

(I)

wherein
R is a linear or branched noncyclic alkyl radical with from 1 to 5 carbon atoms;
(b) 0.9-5.5% by weight of at least one salt selected in the group consisting of NaCl, KCl and mixtures thereof;
(c) 1-10% by weight of at least one ester selected in the group consisting of polyglycerol esters with at least one fatty acid, esters of polyethylene glycol glycerol ethers with at least one fatty acid, and mixtures thereof;
(d) water to 100% by weight.

The amount by weight of (a), (b), (c) and (d) refers to the sum of the weights of (a)+(b)+(c)+(d).
Component (a) is at least one ester of lactic acid and can be independently selected in the group consisting of esters of S-(+)-lactic acid, esters of R-(-)-lactic acid and mixtures thereof.
Component (a) can preferably be selected from methyl lactate, ethyl lactate, isopropyl lactate, n-butyl lactate, isobutyl lactate, isopentyl lactate and mixtures thereof; component (a) can preferably be ethyl lactate.
The above-described lactates, in particular ethyl lactate, dissociate in a basic environment, such as the one present in a wound, generating lactic acid and alcohol, which contribute to a creating a hostile environment for bacterial

proliferation.

The lactic acid esters of the antibacterial composition can preferably be obtained exclusively starting from raw materials of natural origin.

In one embodiment, the antibacterial composition can preferably comprise about 17-45% by weight of component (a).

It has been observed, moreover, that the antibacterial composition comprising an amount less than 2% by weight of component (a) shows no antibacterial activity.

The amount of component (b) in the antibacterial composition can preferably be about 1.0-5.0% by weight.

In one embodiment, component (b) of the antibacterial composition can be a mixture of NaCl and KCl comprising an amount of NaCl greater than or equal to 50% by weight relative to the total weight of component (b). Component (c) is a lipophilic substance that creates an environment which is in itself unfavourable to bacterial proliferation. Furthermore, the polyglycerol esters with fatty acids form complex emulsions with water, which enable an effective transfer of the antibacterial composition onto the absorbent material. The polyglycerol esters with at least one fatty acid can comprise the products of the (trans)esterification of a polyglycerol with at least one vegetable oil and/or with at least one vegetable acid (i.e. the product of hydrogenation of a vegetable oil), and mixtures thereof. Said products, commercially available, are obtained by reacting polymerised glycerol with vegetable acids and/or vegetable oils and/or fatty acids of vegetable origin.

The above-described polyglycerol esters appear in the form of complex mixtures comprising the products of (trans)esterification of polyglycerols, mono-, di- and triglycerides, free glycerol and polyglycerol, free fatty acids and, optionally, salts of fatty acids. The degree of polymerisation of the glycerol, expressed as the average number of glycerol molecules in the compound, can generally range between 3 and 8.

The polyglycerol esters with at least one fatty acid can comprise mixed esters, preferably polyglyceryl-3 cetyl ether olivate/succinate, that is, a monoester of polyglyceryl-3 cetyl ether with a mixture of succinic acid and hydrogenated olive oil.

Component (c) can be selected in the group consisting of esters of polyethylene glycol glycerol ethers with at least one fatty acid. The average degree of ethoxylation of the glycerol can advantageously be comprised between 3 and 9.

The esters of polyethylene glycol glycerol ethers with at least one fatty acid can comprise the products of the (trans)esterification of polyethylene glycol glycerol ethers with at least one vegetable oil and/or with at least one vegetable acid (i.e. product of hydrogenation of a vegetable oil), and mixtures thereof.

In a preferred embodiment, component (c) can be selected in the group consisting of olive oil glycereth-8 esters, coconut oil glycereth-8 esters, polyglyceryl-3 cetyl ether olivate/succinate, polyglyceryl-3 triolivate and mixtures thereof.

Component (d) is water, preferably pharmaceutical grade.

Optionally, and preferably, the antibacterial composition can further comprise an amount less than or equal to 2.0% by weight, preferably 0.1-2.0% by weight of at least one pharmaceutically acceptable auxiliary component (e) selected from preservatives, stabilisers and mixtures thereof. The amount by weight of component (e) refers to the sum of the weights of (a)+(b)+(c)+(d)+(e).

The antibacterial composition can further optionally comprise at least one tissue regeneration promoting agent (f), said tissue regeneration promoting agent preferably being selected in the group consisting of glycoproteins, polysaccharide derivatives, xanthan gums, hyaluronic acid, salts of hyaluronic acid, and mixtures thereof, wherein the amount of the components of the antibacterial composition refer to the sum of the amount of (a)+(b)+(c)+(d)+(e)+(f).

In the preparation of the antibacterial composition, component (b) can advantageously be added in the form of a saline solution with a total salt concentration of about 1-10% by weight.

The antibacterial composition can be prepared with a process comprising:

- mixing, in at least one heated mixer of the type normally used in the pharmaceutical industry, component (b), optionally but preferably as a saline solution, component (c) and optionally component (d), if necessary, by heating the mixture to a maximum temperature less than or equal to about 70°C, thus forming a stable emulsion;
- bringing the temperature of the emulsion to about 40°-50°C;
- adding component (a) and, optionally, auxiliary component (e) and/or the at least one tissue regeneration promoting agent (f) to the emulsion under stirring.

The above-described composition has an antibacterial effect, as it is capable of inhibiting the growth of the bacterial population of both gram-positive and gram-negative bacteria.

It can be advantageously used to prepare an antibacterial material in a process comprising the steps of:

(i) preparing an antibacterial composition as described above, optionally comprising auxiliary component (e);
(ii) optionally preparing a further composition comprising at least one tissue regeneration promoting agent (f);
(iii) optionally mixing the compositions of steps (i) and (ii);

(iv) impregnating an absorbent material with 2-15 g/m$^2$ of the composition of step (i) or step (iii); and
(v) reducing the free water content in the material to a value less than or equal to 0.5% by weight relative to the total weight of the antibacterial material.

The absorbent material of step (iv) can be selected in the group consisting of fabrics, nonwoven fabrics, gauzes, foams, latexes and combinations thereof.
The absorbent material can be selected from materials of natural origin (e.g. cotton, polylactic acid), materials of synthetic origin (e.g. nylon, viscose, polyethylene, polyethylene terephthalate), and combinations thereof.
The at least one tissue regeneration promoting agent comprised in the composition of step (ii) can be selected in the group consisting of glycoproteins, polysaccharide derivatives, xanthan gums, hyaluronic acid and salts thereof, and mixtures thereof.
Step (iv) can be carried out with methods and equipment that are in themselves known in the industry, and can comprise at least one step (iv.a) of immersing the absorbent material in the composition of step (i) or step (iii), spraying the absorbent material with the composition of step (i) or step (iii), and combinations thereof, wherein said at least one step (iv.a) can be carried out under ambient pressure and temperature conditions. The process can optionally, and preferably, comprise a further step (vi) of sterilising the antibacterial material using methods and equipment that are in themselves known in the pharmaceutical industry for the sterilisation of analogous materials.
The antibacterial material obtained from the process is characterised by a water content less than or equal to 0.5% by weight.
The antibacterial material obtained from the process is for topical use and can be applied on skin wounds to prevent and/or inhibit and/or limit bacterial proliferation in skin wounds of a human being or an animal. Therefore, the invention relates to a dressing for skin wounds, comprising (or consisting of) the antibacterial material obtained from the process.
The wound dressing can be selected in the group consisting of gauze compresses, gauze bandages, garments (e.g. socks) and patches; the wound dressing can preferably be an adhesive patch.
In one embodiment, the antibacterial material obtained from the process can be used in the preparation of an adhesive patch comprising at least one layer comprising the antibacterial material.
In particular, the adhesive patch can comprise:

- at least one support layer;
- at least one layer comprising (or consisting of) the above-described antibacterial material disposed on at least one portion of the support layer; and
- at least one adhesive layer disposed on the same surface as the support layer on which the antibacterial material is disposed.

The antibacterial material can be entirely surrounded by the adhesive layer. Alternatively, the adhesive layer can be disposed on opposite sides of a band of antibacterial material.
The adhesive layer can preferably comprise at least one adhesive removable from the skin surface, preferably at least one pressure-sensitive adhesive removable from the skin surface.
If the antibacterial composition and/or antibacterial material do not comprise any tissue regeneration promoting agent, the antibacterial patch can further comprise at least one layer comprising at least one tissue regeneration promoting agent.
The at least one support layer is made with materials normally used in the production of adhesive patches.
The antibacterial patch can be produced using equipment normally used in the industry for this purpose and is therefore not further described herein. The above-described antibacterial patch can have application for topical use to prevent and/or inhibit and/or limit bacterial proliferation in skin wounds of a mammal.
Therefore, the present invention relates to an antibacterial material for topical use in a method for preventing and/or inhibiting and/or limiting bacterial proliferation in skin wounds of a mammal, comprising applying on the wound an antibacterial material or a wound dressing as described above.
The examples that follow are provided solely for illustrative purposes and are not intended to limit the subject matter of the present invention in any way.

**EXAMPLES**

The measurement methods described below were used to determine the properties specified in the entire description and in the claims.

**Example 1**

An antibacterial composition was prepared according to the above-described method, by mixing:

| | |
|---|---|
| an aqueous solution of 2% NaCl by weight | 50g |
| ethyl lactate | 40g |
| polyglyceryl-3 cetyl ether olivate/succinate | 2.0g |
| olive oil glycereth-8 esters | 2.5g |
| natural preservatives | 0.5g |

The composition was sprayed onto a cotton gauze compress in an amount equal to about 5 g/$m^2$ and the water content thereof was reduced to values less than or equal to 0.5% by weight relative to the weight of the gauze. The treated gauze was rested on an agar surface with 10000 CFU/g of gram+ bacteria (S.aureus ATCC6528) and 0.25 mg of water was added progressively. An increase in the inhibition halo was observed with increases in the amount of added water.

An untreated gauze compress creates a minimal inhibition halo (1.1 mm), significantly smaller than the inhibition halo created by the treated gauze sample (1.5 mm).

**Examples 2-4**

Three antibacterial compositions were prepared by mixing, under stirring, at ambient pressure and a temperature of about 70°C, pharmaceutical grade water and the esters specified below, in the proportions shown in table 1. After the emulsion had been formed, the composition was cooled to about 45°C. The saline solutions and lactate were subsequently added. Each composition was sprayed onto a sterile cotton gauze compress in an amount equal to about 10g/$m^2$. The gauze compresses were used to evaluate the antibacterial activity of the composition.

**Table 1**

| | **Ex. 2** | **Ex. 3** | **Ex. 4** |
|---|---|---|---|
| Water | 32 | 34 | 31 |
| NaCl solution 5% p/v | 20 | 20 | 5 |
| KCl solution 5% p/v | 10 | \ | 5 |
| Ethyl lactate | 30 | 40 | 20 |
| Polyglyceryl-3 triolivate | 2 | \ | 0.5 |
| Olive oil glycereth-8 esters | 2.5 | 3 | 3 |
| Coconut oil glycereth-8 esters | 2.5 | \ | 2.5 |
| Polyglyceryl-3 cetyl ether olivate/succinate | \ | 2 | 2 |
| Preservatives | 1 | 1 | 1 |

EVALUATION OF ANTIBACTERIAL ACTIVITY

The test enables an evaluation of the ability of a material to create a barrier effect capable of preventing or strongly limiting the microbial growth of S.aureus and E.coli over time (24 and 48 hours).

Bacterial strains used:

Escherichia coli ATCC 8739
Staphylococcus aureus ATCC 6538

Samples:

Gauze compresses treated with the compositions of examples 2-4
Untreated gauze compresses (negative control)

Test procedure:

The samples and the microorganisms S. aureus and E. coli were prepared for inoculation in accordance with ISO 20743:2008. The microorganisms were inoculated onto the surface of the samples to be tested and the microbial load was assessed at:

- T0 (immediately after inoculation)
- T 24 hours after inoculation
- T 48 hours after inoculation

The inoculation value expressed in CFU/ml is calculated using the formula N=ZxRxV
Wherein:

Z is the mean of the colonies found on the plate;
R is the plate dilution;
V is the volume of the washing broth.

The difference between the maximum and minimum values found in the untreated material was calculated in logs both for time 0 and after 24 hours/48 hours (in order for the test to be valid logmax - logmin ≤ 1.0). Calculate the growth values F and the growth values G where:

$$F = C_t - C_0$$

(in order for the test to be valid F ≥ 1.0)

$$G = T_t - T_0$$

C0 = log of the mean CFU/ml recovered from the untreated material immediately after inoculation;
Ct = log of the mean CFU/ml recovered from the untreated material 24 hours after inoculation;
T0 = log of the mean CFU/ml recovered from the treated material immediately after inoculation;
Tt = log of the mean CFU/ml recovered from the treated material 24 hours after inoculation;

Calculate the value of antibacterial activity A, using the formula A = F-G. In accordance with ISO 20743:2008 a sample is considered to have antibacterial properties if the following conditions are met:

Effectiveness of the antibacterial property Not significant: A <2
Effectiveness of the antibacterial property Significant: 2 ≤ A < 3
Effectiveness of the antibacterial property Strong: A ≥ 3

In the event, however, that the sample cannot be considered to have an antibacterial property (A<2), it can be considered to have a bacteriostatic activity, i.e. capable of preventing or strongly limiting microbial growth. In particular, such activity is demonstrated, in the specific case, if the S.aureus and E.coli load does not increase over time (24 and 48 hours). The results obtained in the test for each sample are shown in the tables below.

**Table 2a**

| | Blank | | Example 2 | |
|---|---|---|---|---|
| | T0 | T24 | T0 | T24 |

(continued)

| | Blank | | Example 2 | |
|---|---|---|---|---|
| S. aureus (CFU/$cm^2$) | Inoculation $3.8\times10^4$ (CFU/$cm^2$) | | | |
| | $4.0\times10^4$ | $9.0\times10^5$ | $1.3\times10^3$ | $1.1\times10^3$ |
| | log=Co=4.60 | log=Ct=5.95 | log=$T_0$=3.11 | log=Tt=3.04 |
| Growth values | F=$C_t$-$C_0$=1.35 | | G=Tt-To= - 0.07 | |
| Antibacteria l activity | A=F-G=1.42 | | | |
| E. coli (CFU/$cm^2$) | Inoculation $3.4\times10^4$ (CFU/$cm^2$) | | | |
| | $3.50\times10^4$ | $9.10\times10^5$ | $1.2\times10^3$ | $9.0\times10^2$ |
| | log=Co=4.54 | log=Ct=5.96 | log=$T_0$=3.08 | log=Tt=2.95 |
| Growth values | F=$C_t$-$C_0$=1.41 | | G=$T_t$-$T_0$= - 0.13 | |
| Antibacteria l activity | A=F-G=1.54 | | | |

The sample of example 2 does not show any significant bactericidal property versus S.aureus and E.coli after 24 hours.

**Table 2b**

| | Blank | | Example 2 | |
|---|---|---|---|---|
| | T0 | T48 | T0 | T48 |
| S. aureus (CFU/$cm^2$) | Inoculation $3.8\times10^4$ (CFU/$cm^2$) | | | |
| | $4.0\times10^4$ | $1.2\times10^6$ | $1.3\times10^3$ | $1.05\times10^3$ |
| | log=Co=4.60 | log=Ct=6.08 | log=$T_0$=3.11 | log=Tt=3.02 |
| Growth values | F=Ct-Co=1.48 | | G=Tt-To= - 0.09 | |
| Antibacteria l activity | A=F-G=1.57 | | | |
| E. coli (CFU/$cm^2$) | Inoculation $3.4\times10^4$ (CFU/$cm^2$) | | | |
| | $3.50\times10^4$ | $1.55\times10^6$ | $1.2\times10^3$ | $9.5\times10^2$ |
| | log=Co=4.54 | log=Ct=6.19 | log=$T_0$=3.08 | log=Tt=2.98 |
| Growth values | F=Ct-Co=1.65 | | G=$T_t$-$T_0$=-0.1 | |
| Antibacteria l activity | A=F-G=1.75 | | | |

The sample of example 2 does not show any significant bactericidal property after 48 hours either.

Since Tt(48) - Tt(24) < 0.1 the S.aureus and E.coli load does not increase over time.

The tested sample is capable of keeping the microbial load of S. aureus and E. coli under control over time; consequently, it is to be considered bacteriostatic.

**Table 3a**

| | Blank | | Example 3 | |
|---|---|---|---|---|
| | T0 | T24 | T0 | T24 |
| S. aureus (CFU/$cm^2$) | Inoculation $2.8\times10^4$ (CFU/$cm^2$) | | | |
| | $2.7\times10^4$ | $1.10\times10^6$ | $1.62\times10^4$ | $1.00\times10^4$ |
| | log=Co=4.43 | log=Ct=6.04 | log=$T_0$=4.21 | log=Tt=4.00 |
| Growth values | F=Ct-Co=1.61 | | G=Tt-To= - 0.21 | |
| Antibacteria l activity | A=F-G=1.82 | | | |

(continued)

| | Blank | | Example 3 | |
|---|---|---|---|---|
| E. coli (CFU/cm$^2$) | Inoculation 4.5×10$^4$ (CFU/cm$^2$) | | | |
| | 4.65×10$^4$ | 8.0×10$^5$ | 9.5×10$^3$ | 3.5×10$^3$ |
| | log=Co=4.67 | log=Ct=5.90 | log=$T_0$=3.98 | log=Tt=3.54 |
| Growth values | F=$C_t$-$C_0$=1.23 | | G=$T_t$-$T_0$=-0.44 | |
| Antibacteria l activity | A=F-G=1.67 | | | |

**Table 3b**

| | Blank | | Example 3 | |
|---|---|---|---|---|
| | T0 | T48 | T0 | T48 |
| S. aureus (CFU/cm$^2$) | Inoculation 2.8×10$^4$ (CFU/cm$^2$) | | | |
| | 2.7×10$^4$ | 1.5×10$^6$ | 1.62×10$^4$ | 1.15×10$^4$ |
| | log=Co=4.43 | log=Ct=6.18 | log=To=4.21 | log=Tt=4.06 |
| Growth values | F=$C_t$-$C_0$=1.74 | | G=$T_t$-$T_0$= - 0.15 | |
| Antibacteria l activity | A=F-G=1.89 | | | |
| E. coli (CFU/cm$^2$) | Inoculation 4.5×10$^4$ (CFU/cm$^2$) | | | |
| | 4.65×10$^4$ | 1.1×10$^6$ | 9.5×10$^3$ | 3.3×10$^3$ |
| | log=Co=4.67 | log=Ct=6.04 | log=$T_0$=3.98 | log=Tt=3.52 |
| Growth values | F=$C_t$-$C_0$=1.37 | | G=$T_t$-$T_0$=-0.46 | |
| Antibacteria l activity | A=F-G=1.83 | | | |

The sample of example 3 does not show any significant bactericidal property, either after 24 hours or 48 hours.

Since Tt(48) - Tt(24) < 0.1 the bacterial load does not increase over time. The tested sample is capable of keeping the microbial load of S. aureus and E. coli under control over time; consequently, it is to be considered bacteriostatic.

**Table 4a**

| | Blank | | Example 4 | |
|---|---|---|---|---|
| | T0 | T24 | T0 | T24 |
| S. aureus (CFU/cm$^2$) | Inoculation 5.1×10$^4$ (CFU/cm$^2$) | | | |
| | 5.2×10$^4$ | 7.0×10$^5$ | 6.8×10$^3$ | 1.9×10$^3$ |
| | log=Co=4.72 | log=Ct=5.85 | log=$T_0$=3.83 | log=Tt=3.28 |
| Growth values | F=Ct-Co=1.13 | | G=Tt-To= - 0.55 | |
| Antibacteria l activity | A=F-G=1.68 | | | |
| E. coli (CFU/cm$^2$) | Inoculation 3.5×10$^4$ (CFU/cm$^2$) | | | |
| | 3.75×10$^4$ | 8.0×10$^5$ | 1.27×10$^4$ | 5.7×10$^3$ |
| | log=Co=4.57 | log=Ct=5.90 | log=$T_0$=4.1 | log=Tt=3.76 |
| Growth values | F=$C_t$-$C_0$=1.33 | | G=Tt-To= - 0.34 | |
| Antibacteria l activity | A=F-G=1.67 | | | |

The sample of example 4 does not show any significant bactericidal property versus S.aureus and E.coli after

24 hours.

**Table 4b**

| | Blank | | Example 4 | |
|---|---|---|---|---|
| | T0 | T48 | T0 | T48 |
| S. aureus ($CFU/cm^2$) | Inoculation $5.1\times10^4$ ($CFU/cm^2$) | | | |
| | $5.2\times10^4$ | $9.0\times10^5$ | $6.8\times10^3$ | $1.65\times10^3$ |
| | log=Co=4.72 | log=Ct=5.95 | log=To=3.83 | log=Tt=3.22 |
| Growth values | $F=C_t-C_0=1.23$ | | G=Tt-To= - 0.61 | |
| Antibacteria I activity | A=F-G=1.84 | | | |
| E. coli ($CFU/cm^2$) | Inoculation $3.5\times10^4$ ($CFU/cm^2$) | | | |
| | $3.75\times10^4$ | $1.2\times10^6$ | $1.27\times10^4$ | $5.6\times10^3$ |
| | log=Co=4.57 | log=Ct=6.08 | $log=T_0=4.1$ | log=Tt=3.75 |
| Growth values | F=Ct-Co=1.51 | | G=Tt-To= - 0.35 | |
| Antibacteria I activity | A=F-G=1.86 | | | |

The sample of example 4 does not show any significant bactericidal property after 48 hours.

Since Tt(48) - Tt(24) < 0.1 the bacterial load does not increase over time and the tested sample is capable of keeping the microbial load of S. aureus and E. coli under control over time; consequently, it is to be considered bacteriostatic.

## Claims

1. An antibacterial composition comprising:

   (a) 2-50% by weight of at least one compound of formula (I)

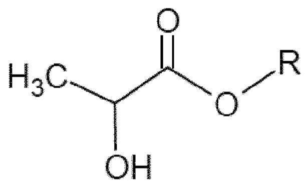

(I)

   wherein
   R is a linear or branched noncyclic alkyl radical with from 1 to 5 carbon atoms;
   (b) 0.9-5.5% by weight, preferably 1.0-5.0% by weight, of at least one salt selected in the group consisting of NaCl, KCl and mixtures thereof;
   (c) 1-10% by weight of at least one ester selected in the group consisting of polyglycerol esters with at least one fatty acid, esters of polyethylene glycol glycerol ethers with at least one fatty acid and mixtures thereof;
   (d) water to 100% by weight.

2. The antibacterial composition according to claim 1, wherein component (a) is selected in the group consisting of methyl lactate, ethyl lactate, isopropyl lactate, n-butyl lactate, isobutyl lactate, isopentyl lactate and mixtures thereof; component (a) is preferably ethyl lactate.

3. The antibacterial composition according to any one of the preceding claims, wherein component (b) is a mixture of NaCl and KCl comprising an amount of NaCl that is greater than or equal to 50% by weight relative to the total weight of component (b).

4. The antibacterial composition according to any one of the preceding claims, wherein the at least one polyglycerol ester with at least one fatty acid comprises products of the (trans)esterification of a polyglycerol with at least one vegetable oil and/or with at least one vegetable acid and the at least one ester of a polyethylene glycol glycerol ether with at least one fatty acid comprises products of the (trans)esterification of polyethylene glycol glycerol ethers with at least one vegetable oil and/or with at least one vegetable acid and mixtures thereof.

5. The antibacterial composition according to any one of the preceding claims, wherein component (c) is selected in the group consisting of olive oil glycereth-8 esters, coconut oil glycereth-8 esters, polyglyceryl-3 cetyl ether olivate/succinate, polyglyceryl-3 triolivate and mixtures thereof.

6. The antibacterial composition according to any one of the preceding claims, further comprising an amount less than or equal to 2.0% by weight, preferably 0.1-2.0% by weight, of at least one pharmaceutically acceptable auxiliary component (e) selected from preservatives, stabilisers and mixtures thereof.

7. The antibacterial composition according to any one of the preceding claims, further comprising at least one tissue regeneration promoting agent (f), preferably selected in the group consisting of glycoproteins, polysaccharide derivatives, xanthan gums, hyaluronic acid, salts of hyaluronic acid and mixtures thereof.

8. A process for preparing an antibacterial material comprising the steps of:

(i) preparing an antibacterial composition as described in any one of claims 1-6;
(ii) optionally preparing a composition comprising at least one tissue regeneration promoting agent (f), as described in claim 7;
(iii) optionally mixing the compositions of steps (i) and (ii);
(iv) impregnating an absorbent material with 2-15 $g/m^2$ of the composition of step (i) or step (iii); and
(v) reducing the free water content in the material to a value less than or equal to 0.5% by weight relative to the total weight of the material.

9. The process according to claim 8, wherein the absorbent material is selected in the group consisting of fabrics, nonwoven fabrics, gauzes, foams, latexes and combinations thereof.

10. The process according to claim 8 or 9, wherein the absorbent material is selected from materials of natural origin, materials of synthetic origin and combinations thereof.

11. An antibacterial material having a water content less than or equal to 0.5% by weight, obtained from the process according to any one of claims 8-10.

12. A dressing for skin wounds comprising the antibacterial material according to claim 11.

13. The dressing for skin wounds according to claim 12, wherein the dressing is selected from the group consisting of gauze compresses, gauze bandages, garments and patches, the wound dressing being preferably an adhesive patch.

14. The dressing for skin wounds according to claim 12, wherein said dressing is a patch, preferably an adhesive patch comprising:

- at least one support layer;
- at least one layer comprising the antibacterial material according to claim 11, disposed on at least one portion of the support layer; and
- at least one adhesive layer disposed on the same surface as the support layer on which the antibacterial material is disposed.

15. The antibacterial material according to claim 11 or the dressing for skin wounds according to any one of claims 12-14 for topical use to prevent and/or inhibit and/or limit bacterial proliferation in skin wounds of a human being or an animal.

## Patentansprüche

1. Antibakterielle Zusammensetzung, umfassend:

(a) 2-50 Gew.- % mindestens einer Verbindung der Formel (I)

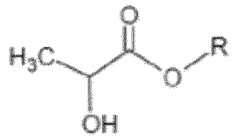

(I)

wobei
R ein linearer oder verzweigter nichtcyclischer Alkylrest mit 1 bis 5 Kohlenstoffatomen ist;
(b) 0,9-5,5 Gew.- %, vorzugsweise 1,0-5,0 Gew.- %, mindestens eines Salzes, ausgewählt aus der Gruppe, bestehend aus NaCl, KCl und Mischungen davon;
(c) 1-10 Gew.- % mindestens eines Esters, ausgewählt aus der Gruppe bestehend aus Polyglycerinestern mit mindestens einer Fettsäure, Estern von Polyethylenglycolglycerinethern mit mindestens einer Fettsäure und Mischungen davon;
(d) 100 Gew.- % Wasser.

2. Antibakterielle Zusammensetzung nach Anspruch 1, wobei Komponente (a) aus der Gruppe bestehend aus Methyllactat, Ethyllactat, Isopropyllactat, n-Butyllactat, Isobutyllactat, Isopentyllactat und Mischungen davon ausgewählt ist; Komponente (a) ist vorzugsweise Ethyllactat.

3. Antibakterielle Zusammensetzung nach einem der vorhergehenden Ansprüche, wobei die Komponente (b) eine Mischung aus NaCl und KCl ist, die eine Menge an NaCl umfasst, die größer oder gleich 50 Gew.- %, bezogen auf das Gesamtgewicht der Komponente (b), ist.

4. Antibakterielle Zusammensetzung nach einem der vorhergehenden Ansprüche, wobei der mindestens eine Polyglycerinester mit mindestens einer Fettsäure Produkte der (Um-)Veresterung eines Polyglycerins mit mindestens einem pflanzlichen Öl und/oder mit mindestens einer pflanzlichen Säure umfasst und der mindestens eine Ester eines Polyethylenglycolglycerinethers mit mindestens einer Fettsäure Produkte der (Um-)Veresterung von Polyethylenglycolglycerinethern mit mindestens einem pflanzlichen Öl und/oder mit mindestens einer pflanzlichen Säure und Mischungen davon umfasst.

5. Antibakterielle Zusammensetzung nach einem der vorhergehenden Ansprüche, wobei Komponente (c) aus der Gruppe bestehend aus Olivenölglycereth-8-estern, Kokosölglycereth-8-estern, Polyglyceryl-3-cetyletherolivate/succinate, Polyglyceryl-3-triolivate und Mischungen davon ausgewählt ist.

6. Antibakterielle Zusammensetzung nach einem der vorhergehenden Ansprüche, ferner umfassend eine Menge von weniger als oder gleich 2,0 Gew.- %, vorzugsweise 0,1-2,0 Gew.- %, mindestens einer pharmazeutisch verträglichen Hilfskomponente (e), ausgewählt aus Konservierungsmitteln, Stabilisatoren und Mischungen davon.

7. Antibakterielle Zusammensetzung nach einem der vorhergehenden Ansprüche, ferner umfassend mindestens ein die Geweberegeneration förderndes Mittel (f), vorzugsweise ausgewählt aus der Gruppe, bestehend aus Glycoproteinen, Polysaccharidderivaten, Xanthangummis, Hyaluronsäure, Salzen von Hyaluronsäure und Mischungen davon.

8. Verfahren zur Vorbereitung eines antibakteriellen Materials, umfassend die Schritte:

(i) Vorbereiten einer antibakteriellen Zusammensetzung wie in einem der Ansprüche 1-6 beschrieben;
(ii) gegebenenfalls Vorbereiten einer Zusammensetzung, die mindestens ein die Geweberegeneration förderndes Mittel (f), wie in Anspruch 7 beschrieben, umfasst;
(iii) gegebenenfalls Mischen der Zusammensetzungen der Schritte (i) und (ii);
(iv) Imprägnieren eines absorbierenden Materials mit 2-15 $g/m^2$ der Zusammensetzung von Schritt (i) oder Schritt (iii); und

(v) Verringern des Gehalts an freiem Wasser im Material auf einen Wert kleiner oder gleich 0,5 Gew.- %, bezogen auf das Gesamtgewicht des Materials.

**9.** Verfahren nach Anspruch 8, wobei das absorbierende Material aus der Gruppe ausgewählt ist, die aus Textilien, Vliesstoffen, Gazen, Schaumstoffen, Latexen und Kombinationen davon besteht.

**10.** Verfahren nach Anspruch 8 oder 9, wobei das absorbierende Material aus Materialien natürlichen Ursprungs, Materialien synthetischen Ursprungs und Kombinationen davon ausgewählt ist.

**11.** Antibakterielles Material mit einem Wassergehalt von weniger als oder gleich 0,5 Gew.- %, das aus dem Verfahren nach einem der Ansprüche 8-10 erhalten wird.

**12.** Verband für Hautwunden, umfassend das antibakterielle Material nach Anspruch 11.

**13.** Verband für Hautwunden nach Anspruch 12, wobei der Wundverband aus der Gruppe ausgewählt ist, die aus Gazekompressen, Mullbinden, Kleidungsstücken und Pflastern besteht, wobei der Wundverband vorzugsweise ein Klebepflaster ist.

**14.** Verband für Hautwunden nach Anspruch 12, wobei der Verband ein Pflaster ist, vorzugsweise ein Klebepflaster, umfassend:

- mindestens eine Trägerschicht;
- mindestens eine Schicht, die das antibakterielle Material nach Anspruch 11 umfasst, das auf mindestens einem Abschnitt der Trägerschicht angeordnet ist; und
- mindestens eine Klebeschicht, die auf der gleichen Oberfläche wie die Trägerschicht angeordnet ist, auf der das antibakterielle Material angeordnet ist.

**15.** Antibakterielles Material nach Anspruch 11 oder Verband für Hautwunden nach einem der Ansprüche 12-14 zur topischen Verwendung zur Verhinderung und/oder Hemmung und/oder Begrenzung der bakteriellen Proliferation in Hautwunden eines Menschen oder eines Tieres.

**Revendications**

**1.** Composition antibactérienne, comprenant :

(a) 2-50 % en poids d'au moins un composé de formule (I)

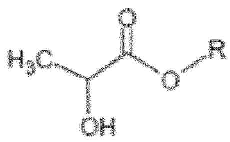

(I)

dans laquelle
R est un radical alkyle non cyclique, linéaire ou ramifié, ayant de 1 à 5 atomes de carbone ;
(b) 0,9-5,5 % en poids, de préférence 1,0-5,0 % en poids, d'au moins un sel choisi dans le groupe constitué par NaCl, KCl et leurs mélanges ;
(c) 1-10 % en poids d'au moins un ester choisi dans le groupe constitué par les esters de polyglycérol avec au moins un acide gras, les esters d'éthers de glycérol de polyéthylène glycol avec au moins un acide gras et leurs mélanges ;
(d) de l'eau jusqu'à 100 % en poids.

**2.** Composition antibactérienne selon la revendication 1, dans laquelle le composant (a) est choisi dans le groupe constitué par le lactate de méthyle, le lactate d'éthyle, le lactate d'isopropyle, le lactate de n-butyle, le lactate d'isobutyle, le lactate d'isopentyle et leurs mélanges ; le composant (a) est de préférence du lactate d'éthyle.

**3.** Composition antibactérienne selon l'une quelconque des revendications précédentes, dans laquelle le composant (b) est un mélange de NaCl et de KCl comprenant une quantité de NaCl supérieure ou égale à 50 % en poids par rapport au poids total du composant (b).

**4.** Composition antibactérienne selon l'une quelconque des revendications précédentes, dans laquelle l'au moins un ester de polyglycérol avec au moins un acide gras comprend les produits de (trans)estérification d'un polyglycérol avec au moins une huile végétale et/ou avec au moins un acide végétal et l'au moins un ester d'un éther de polyéthylène glycol glycérol avec au moins un acide gras comprend des produits de (trans)estérification d'éthers de polyéthylène glycol glycérol avec au moins une huile végétale et/ou avec au moins un acide végétal et leurs mélanges.

**5.** Composition antibactérienne selon l'une quelconque des revendications précédentes, dans laquelle le composant (c) est choisi dans le groupe constitué par les esters glycérothiques-8 d'huile d'olive, les esters glycérothiques-8 d'huile de coco, l'olivate/succinate d'éther cétylique de polyglycéryle-3, le triolivate de polyglycéryle-3 et leurs mélanges.

**6.** Composition antibactérienne selon l'une quelconque des revendications précédentes, comprenant de plus une quantité inférieure ou égale à 2,0 % en poids, de préférence de 0,1-2,0 % en poids, d'au moins un composant auxiliaire (e) pharmaceutiquement acceptable choisi parmi les conservateurs, les stabilisants et leurs mélanges.

**7.** Composition antibactérienne selon l'une quelconque des revendications précédentes, comprenant de plus au moins un agent favorisant la régénération tissulaire (f), de préférence choisi dans le groupe constitué par les glycoprotéines, les dérivés de polysaccharides, les gommes de xanthane, l'acide hyaluronique, les sels d'acide hyaluronique et leurs mélanges.

**8.** Procédé de préparation d'un matériau antibactérien, comprenant les étapes de :

(i) préparer une composition antibactérienne telle que décrite dans l'une quelconque des revendications 1-6 ;
(ii) préparer éventuellement une composition comprenant au moins un agent favorisant la régénération tissulaire (f), tel que décrit dans la revendication 7 ;
(iii) mélanger éventuellement les compositions des étapes (i) et (ii) ;
(iv) imprégner un matériau absorbant avec 2-15 $g/m^2$ de la composition de l'étape (i) ou de l'étape (iii) ; et
(v) réduire la teneur en eau libre dans le matériau à une valeur inférieure ou égale à 0,5 % en poids par rapport au poids total du matériau.

**9.** Procédé selon la revendication 8, dans lequel le matériau absorbant est choisi dans le groupe constitué par les tissus, les tissus non tissés, les gazes, les mousses, les latex et leurs combinaisons.

**10.** Procédé selon la revendication 8 ou 9, dans lequel le matériau absorbant est choisi parmi les matériaux d'origine naturelle, les matériaux d'origine synthétique et leurs combinaisons.

**11.** Matériau antibactérien ayant une teneur en eau inférieure ou égale à 0,5 % en poids, obtenu par le procédé selon l'une quelconque des revendications 8-10.

**12.** Pansement pour plaies cutanées, comprenant le matériau antibactérien selon la revendication 11.

**13.** Pansement pour plaies cutanées selon la revendication 12, dans lequel le pansement est choisi dans le groupe constitué par les compresses de gaze, les bandages de gaze, les vêtements et les patchs, le pansement étant de préférence un patch adhésif.

**14.** Pansement pour plaies cutanées selon la revendication 12, dans lequel ledit pansement est un patch, de préférence un patch adhésif comprenant :

- au moins une couche de support ;
- au moins une couche comprenant le matériau antibactérien selon la revendication 11, disposée sur au moins une partie de la couche de support ; et
- au moins une couche adhésive disposée sur la même surface que la couche de support sur laquelle est disposé le matériau antibactérien.

**15.** Matériau antibactérien selon la revendication 11 ou pansement pour plaies cutanées selon l'une quelconque des revendications 12-14 pour une utilisation topique afin de prévenir et/ou inhiber et/ou limiter la prolifération bactérienne dans les plaies cutanées d'un être humain ou d'un animal.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050019355 A